Europäisches Patentamt

European Patent Office

Office européen des brevets

(19)

(11) Publication number: **0 102 162**
**A1**

(12) # EUROPEAN PATENT APPLICATION

(21) Application number: **83304049.6**

(22) Date of filing: **12.07.83**

(51) Int. Cl.³: **A 61 K 31/185**
**A 61 K 31/235, A 61 K 31/135**
**A 61 K 31/18, A 61 K 31/54**

---

(30) Priority: **14.07.82 US 398848**

(43) Date of publication of application:
**07.03.84 Bulletin 84/10**

(84) Designated Contracting States:
**AT BE CH DE FR GB IT LI LU NL SE**

(71) Applicant: **UNIVERSITY OF GEORGIA RESEARCH FOUNDATION, INC.**
**612 Boyd Graduate Studies Research Center**
**Athens, GA 30602(US)**

(72) Inventor: **Cormier, Milton J.**
**Route 1 Box 34A**
**Hogart Georgia 30622(US)**

(74) Representative: **Lawrence, Peter Robin Broughton et al,**
**GILL JENNINGS & EVERY 53-64 Chancery Lane**
**London WC2A 1HN(GB)**

(54) Use of calmodulin binding drugs for preventing pregnancy.

(57) Certain calmodulin binding drugs are useful as vaginal contraceptives and are preferably provided in the form of a suppository.

The active ingredients can be:
N-PHENYL-NAPHTHYLAMINE, ANTHROYLCHOLINE, NAPHTHALENESULFONAMIDES, PHENOTHIAZINE SULFOXIDES.

One particular calmodulin binding drug can be injected or introduced into the uterus, for instance, with paraffin oil, to prevent preganancy.

EP 0 102 162 A1

Croydon Printing Company Ltd

60/2188/02

- 1 -

## USE OF CALMODULIN BINDING DRUGS FOR
## PREVENTING PREGNANCY

In International Patent Application WO81/03421 we have described methods of preventing conception in a female by introducing a calmodulin binding psychoactive drug into the vagina of the female prior to, or after, sexual intercourse, as well as compositions for use in this method. These compositions generally take the form of a jelly (which may be a true jelly or a cream), a foam (that may be provided as a tablet or an aerosol) or a suppository.

In International Patent Application 8300086 (published after Priority Date of this Application) we have described methods of preventing pregnancy by introducing a calmodulin binding drug into the uterus of a female, generally between intercourse and four days after intercourse. Compositions suitable for preventing pregnancy by this means that are described in that application include foams, jellies and mixtures with paraffin oil.

Some of the active ingredients named in Publication 8300086 are the same as those in Publication 8103421, but some are different. We have now surprisingly found that certain classes of compounds, and specific compounds, that were not specifically proposed for vaginal use in Publication 8103421 are extremely effective as vaginal contraceptives. We have also found that another compound, not mentioned in Publication 8300086, is extremely effective for preventing pregnancy when introduced into the uterus.

The preferred active ingredients for use in the invention are the compounds :

    1.     penfluridol,

    2.     pimozide,

3. trifluoperazine,

4. chlorprothixene,

5. thioridazine,

6. chlorpromazine,

7.. benperidol,

8. haloperidol,

9. clozapine,

10. 8-anilino-1-napthalenesulfontate,

11. 9-anthroylcholine,

12. N-phenyl-1-naphthylamine,

13. N-(6 aminohexyl)-5-chloro-1-napthalenesul-
fonamide,

14. N-(6 aminohexyl)-5-chloro-2-naphthalenesulfonamide

15. N-(6 aminohexy)-5-bromo-2-naphthalenesulfonamide

16. Phenothiazine sulfoxide, or-5-oxide
(Chemical Abstract Registry No. 2232-49-7)

17. Chlorpramazine sulfoxide, or-5-oxide
(Chemical Abstract Registry No. 51887-96-8)

18. 2-Trifluromethyl-N-10 (3'-proprioamino)
phenothiazine sulfoxide, or-5-oxide
(Chemical Abstract Registry No. 1207-71-2)

19. 10-aminopropyl phenothiazine 5 - oxide
(Chemical Abstract Registry No. 51888-10-9) Other phenothiazine sulfoxides can be used since compounds 16 to 19 are merely exemplary of suitable materials.

According to one preferred aspect of the invention a method for preventing conception or pregnancy in a female comprises introducing any of compounds 10 to 19, or other phenothiazine sulfoxides compounds, into the vagina of the female, the drug generally being introduced prior to sexual intercoure and remaining in the vagina during and after intercourse.

According to another aspect of the invention, a contraceptive or pregnancy preventing composition comprises a suppository and includes one or more of compounds 10 to 19 or other phenothiazine sulfoxides.

According to another aspect of the invention a contraceptive pregnancy preventing composition comprises a jelly, foam or other carrier of a type conventionally used in vaginal spermatocidals and includes one or more of compounds 10 to 19, or other phenothiazine sulfoxides.

According to another aspect of the invention, pregnancy in a female is prevented by introducing an effective amount of compound 19 into the uterus of the female, generally between intercourse and four days after intercourse.

According to another aspect of the invention a pregnancy prevention composition comprises compound 19 and a suitable carrier medium for introducing the composition into the uterus, the medium preferably being paraffin oil but optionally being, for instance, a jelly or foam.

Prior to the invention in this case and in the two aforementioned International Patent Publications many forms of contraception are available, including oral contraceptives, mechanical contraceptives, and vaginal contraceptive solutions generally comprising spermatocides. Each form of contraception suffers from undesirable characteristics such as varying effectiveness, discomfort , or physical side affects.

Vaginal contraceptives comprising spermatocidal agents are well known in the prior art in many methods of usage, including jellies and creams (herein referred to as jelly), foams from tablets or aerosols, and suppositories. However, these methods are among the least effective in terms of preventing conception and are basically unsatisfactory as a sole method of contraception.

The present invention primarily comprises the use of certain calmodulin binding drugs as vaginal contraceptives. The introduction of such drugs into the vagina can be accomplished by any of the many commonly available methods currently used in conjunction with spermatocides, such as jelly, foam or suppository means. By substitution of the appropriate amount or concentration of an effective calmodulin binding drug in place of, or in addition to, the spermatocidal agent in any of these means, a form of contraceptive embodying the invention, to be applied in the same manner prior to sexual intercourse, is accomplished.

As stated previously, current forms of vaginal contraceptives are based on the use of spermatocides which are intended to kill the sperm. Generally, the use of such contraceptives by themselves have not proven to be a satisfactorily effective contraceptive method. The present invention does not involve the killing of the sperm, but instead directly and specifically blocks the physiological process of conception and results in greatly improved effectiveness of contraception.

It has been shown in the past years that there is a regulatory protein known as calmodulin, found in all cells of higher organisms and which is the key to the control of a wide variety of physiological processes. We have found that calmodulin is involved in triggering the activation of mammalian spermatozoa, a prerequiste to the fertilisation process. Calmodulin is a calcium binding protein, which means that when calcium is bound to the protein the resulting calcium-protein complex turns on a variety of cellular processes including spermatozoan activation.

Calmodulin binding drugs are drugs that will bind tightly to calmodulin only in the presence of calcium. The binding of these drugs to calmodulin results in the inhibition of calmodulin function.

The use of an effective calmodulin binding drug as a vaginal contraceptive has a number of advantages. First, it is extremely effective since the specific binding of the drug to calmodulin would turn off spermatozoan activiation and thus prevent fertilization. Experimental evidence has demonstrated that the phenothiazine drugs penetrate the spermatozoan membranes within seconds and concentrate in the region of the cell occupied by calmoduline. Second, there will be no expected side effects since the drug would not be used internally and since low concentrations will be very effective as a vaginal contraceptive. Third, the effectiveness of an application may last for hours due to the stability of these drugs.

Additionally, evidence has indicated that calmodulin is also the target protein during ovum activation since this is also a calcium dependent process. Thus if the drug comes into contact with the ovum, fertilisation will not occur. It is seen, therefore, that the present invention may be doubly effective by preventing activiation of both the sperm and the egg.

The most effective drugs for contraceptive purposes can be predicted based on their effectivenss in binding to calmodulin. Compounds 1 to 19, listed above, have been found to be particularly effective.

While a concentration of 0.5% (5 milligrams per milliliter of jelly or other carrier) will be effective in the most effective drugs such as penfluridol or chlorpramazine sulfoxide, less effective drugs will require a higher concentration to achieve similar effectivess. The amount introduced into the vagina is generally about 10mg or greater.

A preferred composition comprises a one-half percent concentration of chlorpromazine sulfoxide in a jelly, introduced into the vagina in sufficient quantity and under known methods prior to sexual intercourse and allowed to remain therein for a period of time, preferably more than a few hours, after intercourse.

Other preferred compositions of the invention comprise suppositories suitable for introduction into the vagina and carrying one or more of compounds 1 to 19, preferably carrying one or more of compounds 10 to 19. The amount of the compound will be selected to be effective, as discussed above.

CLAIMS

1.     A contraceptive composition suitable for application to the vagina and comprising a vaginal carrying medium and an effective amount of a calmodulin binding drug as active ingredient, characterised in that the drug is selected from

1.     8-anilino-1-naphthalenesulfonate,

2.     9-anthroylcholine,

3.     N-phenyl-1-naphthylamine,

4.     N-(6 aminohexyl)-5-chloro-1-naphthalenesulfonamide

5.     N-(6 aminohexyl-5-chloro-2-napthalenesulfonamide

6.     N-(6 aminohexyl)-5-bromo-2-naphthalenesulfonamide,

7.     Phenothiazine sulfoxide, or-5-oxide (Chemical Abstract Registry No. 2232-49-7)

8.     2-Trifluromethyl-N-10 (3' -proprioamina) phenothiazine sulfoxide, or-5-oxide (Chemical Abstract Registry No. 1207-71-2)

9.     10-aminopropyl phenothiazine 5 - oxide (Chemical Abstract Registry No. 51888-10-9)

10.    Chlorpramazine sulfoxide or 5-oxide (Chemical Abstract Registry No. 51887-96-8)

or other phenothiazine sulfoxide.

2.     A composition according to Claim 1, characterised in that it is in the form of a suppository.

3.     A composition according to Claim 1 characterised in that it is in the form of a jelly or a foam.

4.     A pregnancy prevention composition suitable for application to the uterus comprising an effective amount of a calmodulin binding drug and a suitable carrier medium characterised in that the drug is 10-aminopropyl phenothiazine 5 - oxide (Chemical Abstract Registry No. 51888-10-9).

0102162

European Patent
Office

**PARTIAL EUROPEAN SEARCH REPORT**
which under Rule 45 of the European Patent Convention
shall be considered, for the purposes of subsequent
proceedings, as the European search report

Application number

EP 83 30 4049

## DOCUMENTS CONSIDERED TO BE RELEVANT

| Category | Citation of document with indication. where appropriate, of relevant passages | Relevant to claim | CLASSIFICATION OF THE APPLICATION (Int Cl ³) |
|---|---|---|---|
| A | CHEMICAL ABSTRACTS, vol. 81, no. 10, September 9, 1974, page 6, abstract no. 58118h COLUMBUS OHIO (US) U. BREYER et al.: "Tissue accumulation of metabolites during chronic administration of piperazine-substituted phenothiazine drugs". <br><br> & Advan. Biochem. Psychopharmacol. 1974, 9, 167-73 <br><br> * The whole document * | <br><br><br><br><br><br><br><br><br><br>1-5 | A 61 K 31/185 <br> A 61 K 31/235 <br> A 61 K 31/135 <br> A 61 K 31/18 <br> A 61 K 31/54 |
| A | FR - M - 981 (SMITH KLINE & FRENCH LABORATORIES) <br><br> * Claims * | <br><br>1-5 | TECHNICAL FIELDS SEARCHED (Int. Cl ³) <br><br> A 61 K 31/00 |

./.

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-5
Claims searched incompletely:
Claims not searched: 6-9
Reason for the limitation of the search:

Method for treatment of the human or animal body by surgery or therapy (see article 52 (4) of the European Patent Convention).

| Place of search | Date of completion of the search | Examiner |
|---|---|---|
| The Hague | 17-10-1983 | PAUWELS |

**European Patent Office**

**PARTIAL EUROPEAN SEARCH REPORT**

## DOCUMENTS CONSIDERED TO BE RELEVANT

| Category | Citation of document with indication, where appropriate, of relevant passages | Relevant to claim |
|---|---|---|
| E | WO - A - 83/00086 (UNIVERSITY OF GEORGIA RESEARCH FOUNDATION)<br><br>* Claims 10,11,13,14,16,20-24 *<br><br>-- | 1-5 |
| A | WO - A - 81/03421 (UNIVERSITY OF GEORGIA RESEARCH FOUNDATION)<br><br>* Claims *<br><br>---- | 1-3,5 |

CLASSIFICATION OF THE APPLICATION (Int. Cl.)

TECHNICAL FIELDS SEARCHED (Int. Cl.)

EPO Form 1505.3  06.78